Europäisches Patentamt

European Patent Office

Office européen des brevets

(19)

(11) Veröffentlichungsnummer: **0 170 120**
**B1**

(12) **EUROPÄISCHE PATENTSCHRIFT**

(45) Veröffentlichungstag der Patentschrift: **24.10.90**

(21) Anmeldenummer: **85108607.4**

(22) Anmeldetag: **10.07.85**

(51) Int. Cl.⁵: **A 61 F 2/30**, A 61 M 3/00

(54) Vorrichtung und Verfahren zum Mischen und Applizieren von Knochenzement.

(30) Priorität: **11.07.84 DE 3425566**

(43) Veröffentlichungstag der Anmeldung:
**05.02.86 Patentblatt 86/06**

(45) Bekanntmachung des Hinweises auf die
Patenterteilung:
**24.10.90 Patentblatt 90/43**

(84) Benannte Vertragsstaaten:
**AT BE CH DE FR GB IT LI LU NL SE**

(56) Entgegenhaltungen:
CH-A- 480 075
DE-A-2 801 706
DE-A-2 814 353
DE-C- 458 451
DE-U-7 819 584
US-A-4 277 184

(73) Patentinhaber: Draenert, Klaus,
Dr.med.Dr.med.habil.
Gabriel-Max-Strasse 3
D-8000 München 90 (DE)

(72) Erfinder: Draenert, Klaus, Dr.med.Dr.med.habil.
Gabriel-Max-Strasse 3
D-8000 München 90 (DE)

(74) Vertreter: Vossius & Partner
Siebertstrasse 4 P.O. Box 86 07 67
D-8000 München 86 (DE)

Courier Press, Leamington Spa, England.

## Beschreibung

Die Erfindung betrifft eine Vorrichtung und ein Verfahren zum Mischen und Applizieren von Knochenzement.

Bei der heute üblichen Praxis der Gelenkchirurgie erfolgt die Verankerung künstlicher Endoprothesenkomponenten unter Verwendung eines kaltpolymerisierenden "Zwei-Komponenten"-Kunststoffes als Knochenzement, der während der Operation auspolymerisiert und durch seine plastischen Eigenschaften eine Verblockung der Prothesenkomponente im knöchernen Bett bewirkt. Aufgrund seiner physikalischen Eigenschaften Schrumpft der Knochenzement auf die Prothese auf, was zu einem geschlossenen Eingriff zwischen dem Metall oder Kunststoff der Prothese und dem Knochenzement führt.

Als Knochenzemente werden üblicherweise Polymethylmethacrylate (PMMA) verwendet, die aus einem pulverförmigen Perlpolymerisat bestehen, das durch ein flüssiges Monomer öberflächlich angelöst und während der Polymerisation eingelagert wird. In der Mischphase umfließt das Monomer das kugelförmige Perlpolymerisat. Es löst die PMMA-Kugeln oberflächlich an und schließt sie in einem Verbund ein. Die Vorgänge bei der Polymerisation sind erläutert in "Forschung und Fortbildung in der Chirurgie des Bewegungsapparates 1, zur Technik der Zementverankerung", K. Draenert, Art and Science München, 1983.

Ein derartiger Verbund ist beispielsweise mit einem Waschbeton zu vergleichen; wie beim Beton werden beim Mischen Luftblasen eingeschlossen, außerdem kommt es beim Umfließen der PMMA-Kugeln durch das Monomer zu Fülldefekten, die man auch als "Windschattenphänomene" bezeichnet. Zudem kommt es bei den Knochenzementen während der exothermen Polymerisation auch zum Verdampfen der monomeren Flüssigkeit und dadurch zu weiterer Blasenbildung. Die vorstehenden Blasenbildungen stellen den Hauptanteil der Gaseinschlüsse in den Knochenzementen dar.

Die chemische Reaktion der vorstehenden Knochenzemente wird durch eine Startreaktion eingeleitet, für die in der Regel Dibenzoylperoxid durch einen Aktivator, wie p-Aminotoluidin, aktiviert wird und danach die Radikalkettenpolymerisation startet. Diese Polymerisation läuft exotherm ab. Das Monomer selbst ist mit Hydrochinon stabilisiert, manche Knochenzemente erreichen eine weitere Stabilisierung und gleichzeitige Färbung mit Chlorophyll. Die Lagerfähigkeit der monomeren Flüssigkeit läßt sich auch mit Vitamin C stabilisieren.

In der Regel wird das pulverförmige Polymerisat oder Präpolymerisat dem Monomer beigegeben und mit einem Spatel in einer Schale vermischt. In der an die Mischphase anschließenden Verarbeitungsphase wird der Knochenzement meist von Hand, teilweise auch mit einer Spritze in das knöcherne Lager eingebracht, beispielsweise in die Femurmarkhöhle oder in das knöcherne Acetabulum, die für die Verankerung der zementierten Prothesenkomponente vorbereitet sind. Eine derartige Spritze ist beispielsweise in der DE—A—28 01 706 beschrieben.

Durch die Verwendung einer Spritze läßt sich die Knochenzementverankerung im Knochen wesentlich verbessern. Es wurden deshalb auch sog. "Zementkompaktoren" vorgeschlagen, deren Prinzip darin besteht, daß sie den Zement in den abgedichteten Knockenmarkkanal impaktieren, um eine Querverankerung zu erreichen.

Ein derartiger Zementkompaktor ist im "Journal of Bone and Joint", Vol. 65A, Nr. 9, Dezember 83, S. 1335—1338 beschrieben.

Um die störende Geruchsentwicklung und Belästigung durch das verdampfende Monomer zu beseitigen, wurden weiterhin Behälter zum Aufbewahren und Mischen der Pulver- und Flüssigkeitskomponente vorgeschlagen wie beispielsweise in der DE—A—28 01 706 beschrieben. Derartige Behälter stellen ein geschlossenes System dar, sie weisen jedoch in der Praxis alle den Nachteil auf, daß die Vermischung der beiden Komponenten sehr viel schlechter ist, als wenn der Zement wie herkömmlich in der Schale mit einem Spatel angerührt wird.

Andere Vorschläge befassen sich mit dem Problem des Mischens der Zementkomponenten und zielen darauf ab, durch eine bessere Vermischung auch die mechanische Festigkeit der Knochenzemente zu verbessern. Ein derartiger Mischbehälter ist in der DE—A—17 66 334 beschrieben. Dieser Behälter weist eine Kugel auf, die den Behälter in zwei Kammern trennt. Diese Kugel kann eingedrückt werden, so daß sie in einer der beiden Kammern als Rührkörper frei beweglich ist. Dieses Prinzip kann bei zahnärztlichen Amalgamen Verwendung finden, es eignet sich jedoch nicht für die Vermischung von Knochenzementen, da die Kugel nicht mehr aus dem aushärtenden Knochenzement entfernt werden kann und die sich bewegende Kugel zu Laminierungen der immer hochviskoseren Knochenzemente führt. Deshalb kann mit diesem Prinzip keine gleichmäßige Durchmischung von Knochenzementen erreicht werden.

Eine weitere Vorrichtung zum Mischen und Applizieren von Knochenzement ist aus US—A—4 277 184 bekannt. Diese Vorrichtung weist ein zylindrisches Bauteil zur Aufnahme des Knochenzements mit einem verjüngten vorderen Ende mit einer Öffnung auf, die als Applikationsöffnung für den Knochenzement dient. Während des Mischens ist die Öffnung durch einen einschraubbaren Verschluß geschlossen. Innerhalb des Behälters liegt ein Kolben axial verschiebbar an. Zum Mischen des Knochenzements ist ein Mischer vorgesehen, dessen Mischblätter während des Applizierens des Knochenzements in eine Öffnung am Vorderende des Kolbens zurückziehbar sind. Im oberen Teil des Behälters ist eine Öffnung zum Anschluß eines Vakuumschlauchs vorgesehen, durch den die während des Mischen des Knochenzements erzeugten Gase abgeführt werden sollen. Das Applizieren kann mittels Flanschen von Hand wie bei einer Spritze erfolgen, oder es kann ein mechanisches Hilfsmittel in Form einer Quetschzange verwendet werden. Eine Vorkompression des Knochenzements vor der Applikation ist nicht vorgesehen.

Mit den Zementspritzen wird die Auffüllung des knöchernen Bettes in unterschiedlicher Weise verwirklicht. Einerseits erfolgt die Auffüllung von oben nach unten, wie im vorstehenden Artikel im "Journal of Bone and Joint", gezeigt, andererseits auch mit einer langen Düse von unten nach oben, wie in der DE—A—28 14 353 beschrieben. Aus der DE—A—28 14 353 ist ein nach Art einer Injektionsspritze ausgebildetes Operationsinstrument zur Behandlung und Handhabung von Knochenzement bekannt, welches ein Verschlußglied und ein düsenartiges Spritzglied aufweist, die wahlweise am Vorderende des Instruments angebracht werden können. Bei dem Instrument gemäß der DE—A—28 14 353 befindet sich der Knochenzement vor seiner Applikation in einer Patrone, und es kann durch einen Kolben, welcher mittels einer mit einer Klinke zusammenwirkenden Zahnstange bewegt wird, Druck auf den Knochenzement ausgeübt werden. Die Klinke ist schwenkbar gelagert und mittels einer Feder gegen einen Anschlag vorgespannt. Bei Betätigung eines Abzugs wird die Zahnstange mit dem Kolben nach vorn geschoben, bis die Klinke erneut einrastet. Mit diesem Mechanismus läßt sich der Knochenzement derart vorkomprimieren, daß der Druck stetig bis zu einer gewissen Höhe ansteigt und am Ende jedes Hubes der Zahnstange über einen gewissen Zeitraum konstant einstellbar ist. Aufgrund der Zahnteilung der Zahnstange läßt sich aber nicht jeder beliebige Druck einstellen und halten, sondern es sind gewisse Druckintervalle vorgegeben, und die Höhe des mit dem Zahnstangenmechanismus gemäß DE—A—28 14 535 zu erreichenden Drucks ist begrenzt. Bei der Applikation erfolgt bei dem Instrument gemäß der DE—A—28 14 353 ein starker Druckabbau an der Stirnwand der zur Applikation verwendeten langen Düse. Infolgedessen kann der Knochenzement zwar mit einer gewünschten Geschwindigkeit appliziert werden, der Druck ist während der Applikation aber nur in einem eingeschränkten Bereich steuerbar. Ein weiterer Nachteil der Düse ist das sogenannte "Zahnpastaphänomen", d.h. der austretende Knochenzement legt sich wurstförmig aufeinander und führt so zu gravierenden Luft-, Blut- und Markeinschlüssen und Laminierungen, die die mechanische Festigkeit des applizierten Zementes stark beeinträchtigen.

Alle diese Vorschläge hatten letztendlich zum Ziel, die Verankerung der Endoprothesenkomponente im Knochen zu vertiefen und auf diese Weise die Langzeitergebnisse bei Endoprothesenoperationen zu verbessern. Zwar kann durch die Anwendung von Zementspritzen die Verankerung der zementierten Prothese deutlich verbessert werden, es wird jedoch im Vergleich zu dem im Labor hergestellten Knochenzementproben eine immer noch ungenügende mechanische Festigkeit der Knochenzemente erreicht.

Es hat sich nun gezeigt, daß die als Knochenzement verwendeten "Zwei-Komponenten"-Kunststoffe von großen und kleinen Blasen durchsetzt sind, die als locus minoris resistentiae Sollbruchstellen des die Endoprothese umgebenden Zementköchers darstellen. Es sind zwar bereits Versuche bekannt, beispielsweise aus der DE—A—28 14 353, mit entsprechenden Vorrichtungen die Knochenzemente während des Mischen zu verdichten mit dem Ziel, das eingeschlossene Luftvolumen zu verkleinern und damit die mechanische Belastbarkeit der applizierten Knochenzemente zu verbessern. Auch bei den handelsüblichen Spritzen, die teilweise mit einer Einmalkartusche und einem einfachen, von Hand zu bedienenden Spritzkolben arbeiten, wird eine Art Verdichtung des Knochenzementes erreicht. Alle bekannten Spritzen lösen jedoch nicht das Problem, daß der Spritzkolben den den Knochenzement aufnehmenden Zylinder nicht dicht abschließen darf, damit die überstehende Luft nicht in den Zement eingepreßt wird, und sie bieten keine Möglichkeit, den Zementbehälter derartig zu verschließen, daß der Zement hohen Drücken ausgesetzt werden kann. Alle diese Spritzen haben das Problem, soweit ihre Enden verschließbar sind, daß sie an beiden Enden Luft einschließen. Aufgrund der Gesetze der laminaren Strömung wird die den Zement überstehende Luft zentral in die Zementmasse eingeschleust und schwächt gerade den Teil des Zementmantels, der später die Metallprothese umschließt. Die eingeschlossene Luft wird, wie sich gezeigt hat, keinesfalls zur Düse herausgedrückt, sondern aufgrund der laminaren Strömung seitlich in die Zementmasse zurückgedrückt und führt hier zu Sollbruchstellen im Zementmantel. Dies trifft auch auf das Verfahren zur Herstellung von Implantationsmaterialien gemäß EP—A—80 101 583 zu, bei dem die Komponentenmischung vor dem Aushärten in einem nicht dicht abschließbaren Behälter teilweise komprimiert wird. Bei diesem Verfahren läßt sich zudem der erzeugte Druck nicht fein genug dosieren und nicht kontrollieren, und die zur Handhabung und zum Druckaufbau erforderliche Zeit steht bei der Operation, insbesondere wegen des raschen Aushärtens, nicht zur Verfügung. Auch bei dem in DE—U—78 19 584 beschriebenen, abdichtend in einer Tube sitzenden Spritzkolben ist es ebenfalls nicht möglich, die über den Knochenzement überstehende Luft entweichen zu lassen. Außerdem kann mit den Spritzkolben gemäß DE—U—78 19 584 zwar der viskose Knochenzement aus der Tube ausgepreßt werden, eine starke Kompression des Knochenzements ist mit diesem Kolben jedoch nicht möglich, Hinzu kommt, daß das untere Ende der Tube offen ist.

Keine der beschriebenen Spritzen erlaubt es, in kurzer Zeit Drücke aufzubauen und vor allem, diese über eine bestimmte Zeit konstant aufrecht zu erhalten und sie danach in kürzester Zeit wieder abzubauen. Alle bekannten Spritzen mit manueller Kompression erlauben keinen Aufbau hoher Drücke und führen darüberhinaus rasch zur Ermüdung der Muskulatur, so daß ein konstanter Druck nicht gewährleistet ist.

Der Spritzkolben gemäß DE—U—78 19 584 ist somit, wie die Injektionsspritze gemäß der DE—A—28 14 353, allenfalls dazu geeignet, eine laminierungsfreie Injektion des Zementes in den Knochen zu erreichen, nicht jedoch eine wirksame Vorkompression des Zementes, mit der die eingangs erläuterte Blasenbildung wirksam unterdrückt wird. Die mit den bekannten Vorrichtungen von Hand oder einer Übersetzung zu erreichenden Drücke liegen meist weit unter einem Bar (etwa 100 kPa) und sind allenfalls geeignet, die beim Mischen entstandenen großen, eingeschlossenen Luftblasen zu komprimieren. Dies reicht jedoch nicht aus, die mechanische Festigkeit des applizierten Knochenzementes signifikant zu erhöhen.

Ein weiteres Problem besteht darin, daß für den Langzeiterfolg der Operation nicht allein die maximale mechanische Festigkeit des Knochenzements maßgebend ist. Es hat sich nämlich gezeigt, daß es vorteilhaft ist, wenn der Knochenzement im Grenzbereich (Interface) zum Knochen eine gewisse Porosität aufweist. Die poröse Oberfläche des Zements vergrößert die Kontaktfläche erheblich und die histologischen Ergebnisse zeigen, daß durch die Oberflächenvergrößerung der Knocheneinwuchs gefördert wird.

Die Porenbildung kann durch verschiedene Zusätze zu den Knochenzemente über einen weiten Bereich gesteuert werden. Allerdings hat sich gezeigt, daß die Stabilität der Zemente mit zunehmender Porosität abnimmt. Dies bedeutet, daß bei allen Füllsubstanzen die mechanische Festigkeit der Zemente leidet, was, sofern Ersatz durch einwachsende Knochensubstanz ausbleibt, zu einer Schwächung dieser Zemente führt.

Die heute verwendeten Endoprothesen, die Festkörperprothesen, meist aus Metall, darstellen, stellen hohen Anforderungen an die Zementscheide. Sie muß das Eindringen von Körperflüssigkeit und von Granulationsgewebe in das Interface vermeiden. Auf der anderen Seite hat sich, wie vorstehend erläutert, gezeigt, daß Knochen unter geeigneten Bedingungen von der Knochenzementgrenze aus in vorhandene Poren in das Implantat einwächst.

Es hat sich ferner gezeigt, daß bei den herkömmlichen Knochenzementen in der Randzone zum durchbluteten Knochen das flüssige Monomer sehr schnell abfließt bzw. sich verflüchtigt, so daß die Polymerisation des Verbundes Polymer/Monomer gestört ist und der Knochenzement hier besonders brüchig ist. Die Randzone des Zementes zum Knochen ist ferner dadurch gefährdet, daß sich hier, wo keine Blutgefäße vorhanden sind, an der Zementoberfläche Keime besonders günstig entwickeln können. Es ist ferner notwendig, während der Operation die Polymerisationstemperatur des Zements durch zugefügte Füller zu verringern und abzuleiten, um thermische Schäden im Knochen zu vermeiden. Das Einfließen und die Unterschichtung von Blutkoagel und Blut führt zur Laminierung der Knochenzemente und zum Entstehen von Sollbruchstellen der Zementscheide um das Implantat. Durch geeignete Füller in dieser äußeren Schicht kann sowohl eine Blutstillung als auch eine Infektionsprophylaxe, wie auch eine gute Wärmereduktion geschaffen werden. Voraussetzung ist allerdings, daß die mechanische Festigkeit der Zementscheide um das metallene Implantat nicht leidet. Auch resorbierbare Substanzen, wie das in der DE—A—29 05 878 beschriebene Tricalciumphosphat, sind aus diesem Grunde bei Vollschaftimplantaten nur in der äußeren Schicht zum Knochen hin sinnvoll. Ähnliches gilt für Zusätze wie das sog. "bone morphogenetic protein". Es wäre deshalb erfindungsgemäß wünschenswert, den Knochenzement in der Weise zu applizieren, daß um die Metallprothese eine möglichst homogene und mechanisch stabile Zementscheide ausgebildet ist, und daß die Porosität der Kontaktfläche zum Knochen durch Füllstoffe steuerbar ist.

Der Erfindung liegt somit die Aufgabe zugrund, eine Vorrichtung und ein Verfahren zum Mischen und Applizieren von Knochenzement bereitzustellen, mit denen die im Knochenzement eingeschlossenen Blasen verringert werden und die mechanische Festigkeit des Knochenzements erhöht wird. Ferner soll eine gezielt Schichtung des applizierten Knochenzementes möglich sein, insbesondere die Ausbildung einer homogenen Zementscheide mit erhöhter mechanischer Festigkeit um die Prothese und einer äußeren, poröseren Schicht. Das größe Porenvolumen der äußeren Schicht soll den Knocheneinwuchs in den Zementsicherstellen und dadurch die druckaufnehmende Fläche am Knochen vergrößern.

Zur Lösung dieser Aufgabe geht die Erfindung von dem Grundgedanken aus, eine Knochenzementspritze zu schaffen, die einerseits eine vollständige Abdichtung des blasenfreien Knochenzementes während der Vorkompression mit mittleren und hohen Drücken und andererseits eine dosierbare Applikation des Knochenzementes mit steuerbarem Druck zur Auffüllung des knöchernen Lagers ermöglicht. Mit der erfindungsgemäßen Knochenzementspritze ist es auch möglich, bereits vor der Applikation eine Schichtung des Knochenzementes in der Spritze zu erzielen, so daß der applizierte Knochenzement die angestrebte, vorstehend erläuterte Verteilung aufweist.

Die vorstehende Aufgabe wird mit dem Merkmalen der Patentansprüche gelöst.

Die erfindungsgemäße Vorrichtung weist einen entlüftbaren, hermetisch für hohe Drücke gasdicht abdichtbaren Behälter zur Aufnahme des Knochenzements vor seiner Applikation, insbesondere während der Vorkompression auf. Der Behälter ist derart ausbildet, daß der eingeschlossene Zement luftfrei und über eine bestimmte Zeit, vorzugsweise während der gesamten Vorkompression, mit konstantem Druck vorkomprimiert wird. Der auf den Knochenzement wirkende Druck ist exakt einstellbar und steuerbar und es lassen sich hohe Drücke von 2 Bar (etwa 200 kPa) bis zu etwa 20 Bar (etwa 2 MPa) aufbauen.

Der Druck kann durch Druckluft, manuell oder durch einen Motor erzeugt werden und wird durch ein im Behälter bewegliches Ausstoßteil, wie einen Stempel oder Kolben, auf den Zement übertragen. Das Ausstoßteil dichtet gegen den Behälter zumindest zementdicht ab.

Entlüftungsmittel verhindern, daß die den Knochenzement nach dem Einfüllen in den Behälter überstehende Luft durch das Ausstoßteil in den Knochenzement hineingedrückt wird. Auch die gegebenenfalls bereits im Knochenzement durch das Mischen vorhandene Luft kann durch diese Entlüftungsmittel entweichen. Die am distalen Ende des Behälters vorhandene Luft ist durch Lösen der Verschlußkappe abführbar.

Bei der erfindungsgemäßen Vorrichtung wirkt im Betrieb das bewegliche Ausstoßteil mit Drükken zwischen etwa 100 kPa und 2 MPa in geschlossenem Kontakt auf die aushärtende Knochenzementmasse ein. Auf diese Weise wird während der Polymerisation die aufgrund der exothermen Reaktion ohne Komprimierung auftretende Monomerverdampfung weitgehend unterbunden. Zusätzlich werden die Luftblasen aus der Durchmischungsphase stark komprimiert, das Perlpolymerisat besser umschlossen und Fülldefekte vermieden. Auf diese Weise entsteht zwischen dem Ende der Mischphase und dem ersten Drittel der Verarbeitungsphase ein sehr kompakter Verbundwerkstoff.

Durch den erhöhten Druck bei der Vorkompression laufen ferner die chemischen Reaktionen in Knochenzement vollständig ab. Dies hat einen extrem niedrigen Restmonomergehalt zur Folge. Die höhere Fülldichte und die vollständigere chemische Polymerisation erhöhen die mechanische Festigkeit des applizierten Knochenzements wesentlich. Weitere Vorteile der erfindungsgemäßen Vorrichtung liegen in der einfachen Handhabung und der sicheren, gezielten Applikation des Knochenzements.

Zur Kontrolle des Druckes während der Vorkomprimierung und der Applikation ist im druckbeaufschlagten Bereich der Vorrichtung vorzugsweise eine Druckmeßvorrichtung, wie ein flacher Zylinder mit Spiralfeder und einer farbigen Kalibrierung angeordnt, die durch ein sterilisierbares Fenster durch Farbringe den jeweils herrschenden Druck anzeigt, wobei jeweils bestimmte Farbmarkierungen für den korrekten Druck in der Kompressions- und Applikationsphase vorgesehen sein können. Die Druckmeßvorrichtung kann auch hinter dem Ausstoßteil angeordnet und am hinteren Ende der Spritze ablesbar sein.

Der Druck auf den Knochenzement wird vorzugsweise dadurch erzeugt, daß komprimierte Gase, vorzugsweise Druckluft, über eine Zuführung in eine Kompressionskammer eingeleitet werden und auf das hintere Ende des Ausstoßteiles, wie eines Druckstempels oder Kolbens ein wirken, dessen Umfang gegen eine Gehäuse, wie einen Druckzylinder abgedichtet ist und der unter der Einwirkung des Drucks nach vorne beweglich ist. Der auf das Ausstoßteil einwirkende Druck ist mittels mindestens eines Ventils steuerbar. In der Kompressionsphase wird vorzugsweise ein fester Druck eingestellt, dessen Wert von der Viskosität des Knochenzements abhängt. In der Applikationsphase, ist der Druck mittels des Ventiles oder der Ventile stufenlos dosierbar. Das vordere Ende des Ausstoßteils ragt in den Zementbehälter hinein und ist gegen dessen Innenwand derart abgedichtet, daß allenfalls Luft oder Gas, nicht aber Zement entweichen kann, zumindest nachdem es mit dem Knochenzement in Kontakt steht.

Bei der Gaskompression des Knochenzements beträgt der Leitungsdruck vorteilhafterweise 500 kPa bis 1,5 MPa, vorzugweise etwa 800 kPa bis 1 MPa. Der Druck am vorderen Ende des Knochenzementbehälters beträgt dann etwa 300 bis 600 kPa.

Bei einer weiteren Ausführungsform wird der Druck auf den Knochenzement manuell oder motorgetrieben durch Drehen einer Gewindestange erzeugt, nachdem das Ausstoßteil den Zementspiegel erreicht hat. Hierdurch läßt sich im Knochenzement ein Druck von etwa 400 bis 800 kPa, vorzugsweise etwa 500 bis 600 kPa, aufbauen. Der Zementausstoß erfolgt bei dieser Ausführungsform ebenfalls manuell oder motorgetrieben durch Vorwärtsschieben des Ausstoßteils.

Die vorstehenden Druckwerte sind jeweils als Überdruck gegenüber Atmosphärendruck zu verstehen.

Um zu verhindern, daß die nach dem Einfüllen und Mischen des Zements über den Zement überstehende Luft während der Kompression in den Knochenzement hineingedrückt wird, sind Entlüftungsmittel vorgesehen. Vorzugsweise sind diese Entlüftungsmittel als mindestens eine axiale Längsnut oder mehrere, in Axialrichtung beabstandete Öffnungen, beispielsweise mehrere Reihen von in Axialrichtung angeordneten Öffnungen oder als Ventile im Behälter ausgebildet. Um eine exakte Anpassung der Entlüftungsmittel an die Füllhöhe des Zementes zu ermöglichen, ist vorzugsweise eine auf dem Behälter verschiebbare äußere Hülse oder Manschette vorgesehen, mit der ein Teil der Entlüftungsmittel dicht verschließbar ist.

Bei einer weiteren Ausführungsform sind die Entlüftungsmittel als Lamellenkörper ausgebildet, der auf das vordere Ende des Ausstoßteils aufgesetzt ist. Der Lamellenkörper oder Lamellenzylinder weist mindestens eine, vorzugsweise zwei bis vier, besonders bevorzugt drei flexible Lamellen auf, deren Querschnitt etwa dem Innendurchmesser des Zementbehälters entspricht. Während der Vorwärtsbewegung des Ausstoßteils kann die den Zement überstehende, eingeschlossene Luft außen an den Lamellen vorbeistreichen, mit Erreichen des Zementniveaus und Druckaufbau dichten die Lamellen jedoch zumindest zementdicht gegen den Behälter ab. Gegebenenfalls können die Lamellen in Kontakt mit dem Zement auch luftdicht gegen die Innenfläche des Behälters abdichten. Auf diese Weise läßt sich besonders einfach eine Entlüftung vor der Kompression erzielen. Der Lamellenkörper besteht vorzugsweise aus Teflon® (Warenzeichen von Du Pont de Nemours and Co. für Fluorkohlenstoffe aus Polytetrafluoräthylen und Polyperfluoräthylenpropylen).

Während der Kompressionsphase ist der Behälter an seinem vorderen, distalen Ende mittels einer Verschlußkappe druckdicht abgeschlossen. Die Verschlußkappe wird vorzugsweise mittels eines Bajonettverschlusses mit steigendem Gewinde aufgeschraubt, so daß der geschlossene Behälter Drücken bis zu etwa 2 MPa widerstehen kann. Vorteilhafterweise ist der Zementbehälter, insbesondere um sterile Bedingungen zu erleichtern, als Einwegbehälter, wie Einmalkartusche ausgebildet, die mittels eines Schnellverschlusses beispielsweise eines Bajonettverschlusses, am Gehäuse befestigbar ist. Der Bajonettverschluß kann mit einer Überwurfmutter gesichert werden.

Um die Handhabung und die Applikation zu erleichtern, weist die Knochenzementspritze vorzugsweise die Form einer Pistole auf, wobei der Zementbehälter, der Kolben und die Kompressionskammer im wesentlichen den "Lauf" der Pistole ausbilden und die Ventilsteuerung von dem im wesentlichen senkrecht zum "Lauf" angeordneten Griffteil aufgenommen wird.

Die Betriebsweise der erfindungsgemäßen Vorrichtung sieht dabei vorzugsweise folgendermaßen aus: Der Knochenzement wird in den Einwegbehälter eingefüllt, der an seinem vorderen, distalen Ende durch Aufschrauben der Verschlußkappe mittels eines Bajonettverschlusses mit steigendem Gewinde verschlossen wird; der Behälter hält mindestens Drücken bis zu etwa 2 MPa stand. Die Kappe wird zunächst zur distalen Entlüftung locker aufgesetzt. Der zuvor in einer Schale angerührte Zement wird in seiner niedrigviskosen Phase in den Behälter eingegossen, anschließend wird die Kappe festgezogen und der zylinderförmige Behälter über das Ausstoßteil geschoben und in einem Bajonettverschluß am Pistolenlauf aufgesetzt. Das sich nach Druckaufbau im Behälter vorschiebende Ausstoßteil drückt die über den Zement überstehende Luft durch die seitlich bis in Höhe des Zementspiegels angeordneten Entlüftungslöcher heraus, die zuvor beispielsweise durch eine außen verschiebbare Manschette auf die variable Zementmenge eingestellt worden sind, oder die überstehende Luft streicht an den Lamellen des Lamellenkörpers vorbei nach hinten.

Bei der Ausführungsform mit Entlüftungsöffnungen im Behälter ist das kolbenförmige Ausstoßteil an seinem vorderen Ende mittels eines Schiebers mit Dichtringen gegen den inneren Umfang des Zementbehälters hermetisch dicht abgeschlossen. Bei der Ausführungsform mit Lamellenkörper dichten die Lamellen ebenfalls zumindest zementdicht gegen den inneren Umfang des Zementbehälters ab, sobald das Ausstoßteil beim Vorwärtsbewegen das Zementniveau erreicht und Druck aufgebaut wird.

Durch den aufgebauten Druck wird die Zementsäule in schlüssigen Kontakt mit der Verschlußkappe gebracht, und der Zement wird etwa vom Ende der ersten Minute nach Polymerisationsbeginn an unter starke Kompression gesetzt. Die Kompressionsdauer beträgt je nach Zementsorte etwa 1,5 bis 5 Minuten nach Polymerisationsbeginn, bei vorzugsweise konstantem Druck, wobei die Kompressionsdauer bei hochviskosen Zementen geringer ist. Die zur Verhinderung der Blasenbildung erforderlichen Drücke betragen je nach Viskosität der verwandten Knochenzemente mindestens etwa 0,2 bis 2 MPa. Soweit die Hausleitungen nur für Drücke bis 0,6 oder 1,0 MPa ausgelegt sind, können Druckverstärker eingebaut werden.

Während der Vorkompression wird zur Konstanthaltung des Drucks das Ausstoßteil mechanisch oder in anderer Weise arretiert. Bei der druckluftbetätigten Ausführungsform wird durch Betätigung eines der Ventile die Kompressionskammer geschlossen, und in dieser Position kann das Ventil durch einen Feststellregler temporär fixiert werden. Am Ende der Vorkomprimierungsphase wird die Druckkammer entlastet, vorzugsweise durch Betätigung von zwei Ventilen, einem Entriegelungsventil und einem Entlüftungsventil. Dadurch ist es möglich, die Verschlußkappe des Einwegbehälters abzunehmen. Danach wird der Knochenzement mittels des Einlaßventiles mit fließend dosierbaren Drücken in den Knochen eingepreßt. Di erfindungsgemäße Vorrichtung bietet aufgrund der möglichen Abdichtung der Femurmarkhöhle auch alle Möglichkeiten des Impaktierens.

Erfindungsgemäß kann die Steuerung des auf den Knochenzement einwirkenden Druckes auf verschiedene Weise erfolgen; es ist wesentlich, daß während der Vorkomprimierung ein höherer, vorzugsweise konstant einstellbarer oder steuerbarer Druck auf den Zement einwirkt, während bei der Applikation der Zement mit niedrigerem, stufenlos dosierbarem Druck ausgestoßen wird. Bei einer Ausführungsform der Erfindung erfolgt die Drucksteuerung mittels mindestens zweier Ventile oder Schieber, die vorzugsweise jeweils zwei Schaltstellungen aufweisen. Durch Betätigen und Arretieren des ersten Ventils wird zunächst in der Vorkompressionsphase die Kompressionskammer unter hohen Druck gesetzt, wobei dieser Druck vorteilhafterweise an der Druckquelle einstellbar und vorgegeben ist.

Mit dem zweiten Ventil kann das erste Ventil entriegelt, die Kompressionskammer geschlossen und die Entlüftungsleitung geöffnet werden. Zur Entlastung der Kompressionskammer werden beide Ventile betätigt. Zur Dosierung des Druckes bei der Applikation werden die beiden Ventile in eine derartige Stellung gebracht, daß sowohl Zuleitung, als auch Einlüftungsleitung geöffnet sind, wobei mindestens eine der beiden Leitungen durch eines der beiden Ventile dosiert zu öffnen ist; vorzugsweise erfolgt die Dosierung mittels des als Einlaßventil ausgebildeten ersten Ventils. Bei einer alternativen Ausführungsform kann eines der beiden Ventile, vorzugsweise das erste Einlaßventil, auch als Druck reduzierventil ausgebildet sind. Die Entlüftung kann auch mittels eines dritten Ventils oder eines Entlüftungshebels oder -knopfes erfolgen.

Die erfolderlichen Funktionen bei der Drucksteuerung Konnen erfindungsgemäß auch mittels eines einzigen Ventiles erreicht werden, das mindestens drei Schaltstellungen aufweist, in denen erstens ein einstellbarer, hoher Druck in der Kompressionskammer, zweitens eine Entlastung der Kompressionskammer und drittens ein fein dosierbarer Druck erzielt werden.

Die Schichtung des Zementes kann dadurch erreicht werden, daß im Behälter der erfindungsgemäßen Vorrichtung zwei oder mehrere verschieden angerührte Zementportionen übereinandergeschichtet werden. Die Zementmasse mit Füllern wird als unterste Schicht im Behälter aufgeschichtet, beispielsweise in einem Verhältnis von etwa einem Drittel oder der Hälfte der Gesamtmasse. Darüber wird eine gesondert angerührte Zementmasse geschichtet, die homogen ist und hohe Dichte aufweist. Der Zementbehälter kann in der vorstehend geschilderten Weise am unteren Ende verschlossen und mit dem offenen Ende am "Lauf" der Spritze befestigt werden. Die danach folgende Verdichtung durch Komprimierung des Zementes führt nicht zu einer Durchmischung der Schichten.

Die vorstehend erläuterte angestrebte Schichtung des Zementes im Knochen kann in einfacher Weise dadurch erreicht und in ihrer Ausbildung gesteuert werden, daß die Öffnung des zylindrischen Zementbehälters als offener Kegelstumpf ausgebildet ist, wobei das Verhältnis des Durchmessers der Spritzenöffnung an deren vorderem Ende zum Durchmesser des Zylinderbehälters in definierter Weise aufeinander eingestellt werden. In Abhängigkeit von der Viskosität des Zementes und den gewünschten Schichtdicken beträgt das Verhältnis des Durchmessers des Zementbehälters zum vorderen Durchmesser der Öffnung etwa 2:1 bis 4:3, vorzugsweise 3:2. Besonders vorteilhaft ist es, die vordere Öffnung durch eine Blende, beispielsweise aufsetzbare Kappen, steuerbar zu machen; dadurch kann die Applizierung besonders variabel den jeweiligen Verhältnissen angepaßt werden. Auch durch die Wahl des Neigungswinkels des Kegelstumpfes läßt sich die Schichtung gegebenenfalls geeignet beeinflussen.

Das distale Ende des Behälters kann auch als nicht rotationssymmetrischer Stumpf ausgebildet sein, dessen Mantel auf zwei Seiten abgeflacht ist und eine ovale Öffnung aufweist. Dies erleichtert die gezielte Applikation entsprechend dem offenen Ende des Femurs.

Die Schichtung des Zementes beim Applizieren erfolgt in der Weise, daß die untere Portion mit den Füllstoffen sich in breiter Masse am proximalen Ende des Knochenmarkkanals verankert, während die zweite homogene und dichte Portion zentral durch die Masse der ersten nach vorne und nach unten geschoben wird. Die Metallprothese wird danach in der Mitte der Zementmasse eingeschoben, so daß sich infolge der laminaren Schichtung die zweite Zementportion exakt dem Metall anlegt. Diese Schichtung kann in Abhängigkeit von der Spritzenöffnung variiert werden. Z.B. kann durch Wahl einer sehr weiten oder sehr engen Öffnung ein dicker äußerer Mantel und eine dünne, dichte Schicht innen bzw. ein dünner Mantel und eine starke dichte Schicht hergestellt werden. Hierbei kann man sich der Viskosität des Zementes und dem Prothesentyp anpassen. Die Vorteile einer Steuerung der Öffnung durch Blenden liegen insbesondere in der Möglichkeit, sich auch der Form des proximalen Femurs anpassen zu können.

Die vorstehenden Ergebnisse sind sicher reproduzierbar, auch in den Fällen, in denen um die Prothese ein Prothesenstrumpf zur Stabilisierung der Zementscheide eingeführt wird. In diesen Fällen wird die Zementspritze zentral im Strumpf aufgesetzt und der Zement eingespritzt. Auch hierbei ergibt sich eine exakte Schichtung in einen grob porösen äußeren Mantel und eine dichte innere Scheide. Die Querverblocking der Zementmasse durch in transversale Volkamm-'sche Kanäle eindringende plastische Zementmassen wird durch Applikation der zum Teil niedrigviskosen Füllstoffe nicht beeinträchtigte, denn bei Eindringen des dichteren Zementes in die Volkmann'schen Kanäle wird diese Füllsubstanz wie ein Mantel abgestreift, so daß die Stabilität nicht verringert wird.

Statt in einem Behälter können die verschiedenen Zemente auch in zwei getrennten Plastikzylindern im geschlossenen System angerührt werden. Die beiden Plastikzylinder werden dabei mittels eines Adapters in Axialrichtung miteinander gekoppelt, z.B. mittels eines Schnellverschlusses mit steigendem Bajonettgewinde, wodurch einerseits die Entlüftung, andererseits die Druckdichtigkeit des Systems ermöglicht wird.

Für manche Füllsubstanzen kann eine Schichtung erfindungsgemäß auch besonders vorteilhaft in anderer Weise erzielt werden. Insbesondere für alle Füllsubstanzen, die eine größere Dichte als der Knochenzement aufweisen, wie Röntgenkontrastmittel, gesinterte Apatite oder Apatite hoher Dichte, führt eine koaxiale Rotation zu einer idealen Schichtung bereits in der Spritze selbst. Die homogene Zementmasse nimmt dabei während der Rotation die Zentrale Portion und die schwereren Füllerpartikel augrund der Zentrifugalkraft die äußeren Schichten im Zementbehälter ein. Im Rahmen der Erfindung ist es auch möglich, eine Zementspritze mit rotierendem Zementbehälter vorzusehen, ohne daß der Zement im Behälter vorkomprimiert wird.

In einer bevorzugten Ausführungsform der erfindungsgemäßen Vorrichtung ist die Zementspritze sowohl für die hohen Vorkomprimierung der Knochenzemente, als auch für die koaxiale Rotation geeignet. Kompression und Rotation können besonders vorteilhaft jeweils durch komprimierte Gase, wie Druckluft, hervorgerufen werden. Dieses Vorgehen hat darüberhinaus den Vorteil, daß der Zement in der üblichen Weise auf einmal angemacht werden kann. Der Zementbehälter mit der eingefüllten Masse wird an seinem vorderen Ende verschlossen und danach in der üblichen Weise auf den Applikator aufgesteckt und die Zementmasse durch Betätigung und Verriegelung des Einlaßventiles unter Druck gesetzt.

Durch Schließen des zweiten Ventiles bleibt die Kompressionskammer unter voller Kompression. Nunmehr wird der Zementbehälter unter konstanter Kompression durch ein Umwegventil vorzugsweise durch weiteres Betätigen des Einlaßventiles, mittels einer turbinengetriebenen Drehvorrichtung in axiale Rotation versetzt, ähnlich einer Druckluftbohrmaschine. Als Antrieb für die Drehvorrichtung kann auch ein Elektromotor vorgesehen sein, dabei ist es aber schwieriger, sterile Bedingungen einzuhalten.

Mit dieser Ausführungsform der erfindungsgemäßen Vorrichtung kann der Knochenzement in seiner niedrigviskosen Phase sowohl vorkomprimiert als auch zentrifugiert werden. Die Drehachse ist dabei koaxial zur Achse des Zementbehälters; durch diese koaxiale Rotation wird eine Entmischung des Zementes, wie sie bei exzentrischer Rotation beispielsweise in einer Laborzentrifuge stattfinden würde, vermieden. Auf diese Weise wird erreicht, daß die schweren Füllerpartikel nach dem Rotieren in dem äußeren Schichten anzutreffen sind, aufgrund des laminaren Fließverhalten des Knochenzementes bildet der die Füllerpartikel enthaltende Zement auch nach dem Applizieren die zum Knochen weisende äußere Schicht aus. Die zentrale Portion in der Spritze wird durch die homogene und dichte Zementmasse ausgebildet, die nach dem Applizieren unmittelbar um die Metallprothese zu liegen kommt.

Die Erfindung wird nachstehende anhand der Zeichnung näher erläutert. Es zeigen:

Fig. 1 eine Gesamtansicht der montagefertigen erfindungsgemäßen Vorrichtung,

Fig. 2 den Querschnitt einer ersten Ausführungsform der erfindungsgemäßen Vorrichtung,

Fig. 3 den Querschnitt einer weiteren Ausführungsform der erfindungsgemäßen Vorrichtung mit gasdruckgetriebener Drehvorrichtung,

Fig. 4 eine teilweise geschnittene Ansicht einer manuell betätigbaren Ausführungsform der erfindungsgemäßen Vorrichtung, und

Fig. 5 einen Teil der Vorrichtung von Fig. 4.

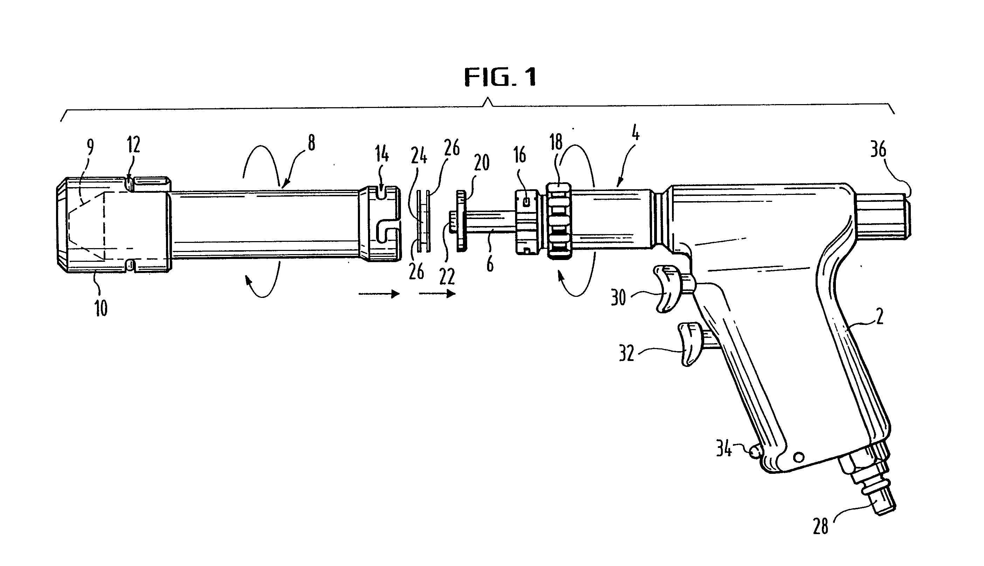

Die in Fig. 1 dargestellte druckluftbetätigte Knochenzementpistole weist einen Handgriff oder Haltegriff 2, ein Gehäuse 4, ein Ausstoßteil 6, einen Knochenzementbehälter 8, wie eine Kartusche, mit sich konisch verjüngendem vorderen Ende 9 und eine Verschlußkappe 10 auf. Der Behälter 8 und die Verschlußkappe 10 sind durch eine Kupplung, wie einen Bajonettverschluß 12 miteinander verbunden. Am anderen Ende des Behälters 8 ist ein Schnellverschluß 14 in Form eines Bajonettgewindes vorgesehen, das bei der Montage der Knochenzementpistole in Stifte 16 am vorderen Ende des Gehäuses 4 einrastet und den Behälter 8 mit dem Gehäuse 4 verbindet. Auf dem Gehäuse 4 ist ferner einer drehbare Bajonettsicherung 18 vorgesehen.

Das Ausstoßteil 6 weist an seinem vorderen Ende einen Schieber 20 mit einem kurzen Zapfen 22 mit kleinerem Durchmesser auf. Der Zapfen 22 paßt in eine innere Öffnung eines Lamellenkörpers 24 mit zwei flexiblen Lamellen 26. Der Lamellenkörper 24 ist als Wegwerfteil ausgebildet.

Am rückwärtigen Ende des Haltegriffs 2 ist ein Druckluftanschluß 28 zur Verbindung mit einer (nicht dargestellten) Druckluftquelle vorgesehen. Ferner sind am Haltegriff 2 ein als Dosierventil ausgebildetes Druckeinlaßventil 30, ein Entriegelungsventil 32 zum Entriegeln des Druckeinlaßventils 30 und ein Entlüftungsknopf 34 angeordnet. Ferner weist der Haltegriff 2 in Verlängerung des Gehäuses 4 eine farbige Druckanzeige 36 auf.

Die Knochenzementpistole gemäß Fig. 1 ist in montagefertigem Zustand. Zunächste wird nach dem Ansetzen des Knochenzements durch Zugabe des Pulvers zum Monomer und Mischen bei mäßiger Rührgeschwindigkeit der Knochenzement in den durch die Verschlußkappe 10 geschlossenen Behälter 8 eingefüllt. Nach Aufsetzen des Lamellenkörpers 24 auf den Schieber 20 des Ausstoßteils 6 wird die Knochenzementpistole durch Arretieren des aus Bajonettgewinde 14 und Stiften 16 bestehenden Schnellverschlusses montiert. Das distale Ende des Behälters wird bei locker aufgesetzter Kappe 10 durch kurzes Betätigen des Ventils 30 entlüftet, und die Kappe 10 danach festgezogen. Danach wird das Druckeinlaßventil 30 betätigt und eingerastet, und die über den Druckluftanschluß 28 zugeführte Druckluft drückt das Ausstoßteil 6 mit dem aufgesetzten Lamellenkörper 24 im Behälter 8 nach vorne, wobei die überstehende Luft zwangsweise an den flexiblen Lamellen 26 vorbeistreicht. Wenn der Lamellenkörper 24 in schlüssigem Kontakt mit dem Zement im Behälter 8 steht, dichten die Lamellen 26 zumindest zementdicht gegen die Innenfläche des Behälters 8 ab. Hierzu weist der Lamellenkörper 24 vorzugsweise drei Lamellen 26 auf, um eine vollständige Zementdichtigkeit zu gewährleisten. Der Knochenzement wird etwa von der zweiten bis zur vierten Minute ab Polymerisationsbeginn mit konstantem Druck vorkomprimiert, der bei einem Druck von etwa 800 kPa am Anschluß 28 am distalen Ende des Behälters 8 etwa 300—500 kPa beträgt.

Etwa fünf Minuten nach Polymerisationsbeginn wird das Innere der Zementpistole durch Drücken des Entriegelungsventils 32 und des Entlüftungsknopfes 34 belüftet, und die Verschlußkappe 10 kann nach Druckabbau entfernt werden. Durch erneutes Betätigen des Druckeinlaßventils 30 kann der Knochenzement appliziert werden. In diesem Fall wird das Druckeinlaßventil 30 nicht eingerastet, und der Applikationsdruck kann stufenlos dosiert werden. Die Drücke betragen hierbei vorzugsweise etwa 100 bis 200 kPa, wobei der Druck während der Applikation variiert, z.b. langsam erhöht werden kann.

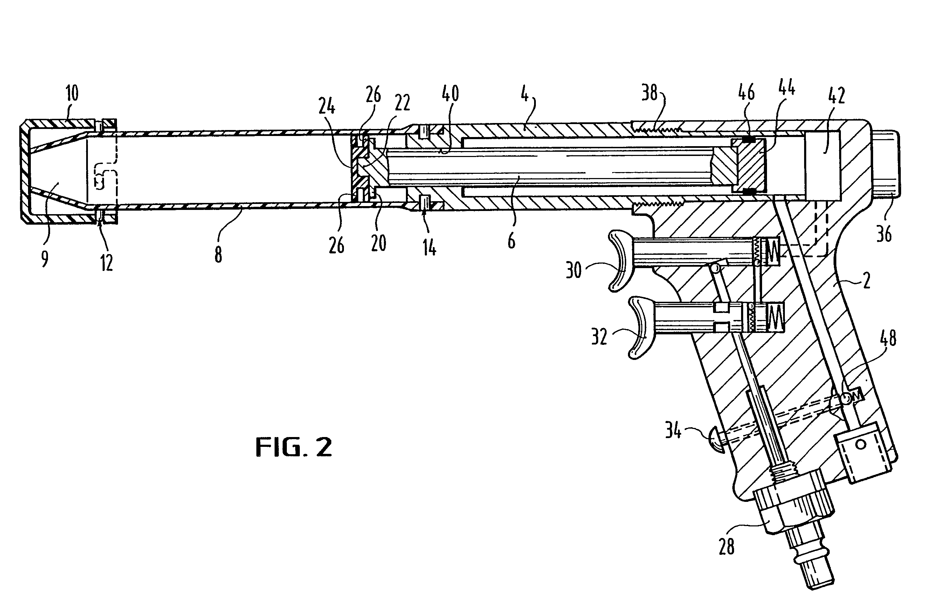

Die in Fig. 2 im Schnitt dargestellte Vorrichtung entspricht im wesentlichen der Vorrichtung gemäß Fig. 1 im montierten Zustand.

Das Gehäuse 4 ist mittels eines Gewindes 38 mit dem Haltegriff 2 verschraubt und weist an seinem vorderen Ende eine Kolbenführung 40 für das Ausstoßteil 6 auf.

Im Innern des Haltegriffs 2 ist hinter dem Gehäuse 4 eine Kompressionskammer 42 angeordnet, die durch Betätigen des Druckeinlaßventils 30 mit einem über den Druckluftanschluß 28 zugeführten komprimierten Gas oder Fluid, wie Druckluft, gefüllt wird. Das Ausstoßteil 6 weist an seinem hinteren Ende einen hinteren Schieber 44 auf. Der Schieber 44 weist eine Abdichtung, beispielsweise einen Abdichtung 46 auf, der gasdicht an den Innenwand des Gehäuses 4 anliegt, wodurch der Kompressionsraum 42 vom hinteren Ende des Ausstoßteils 6 druckdicht abgeschlossen wird. Ferner ist ein Entlüftungsventil 48 vorgesehen, über das durch Betätigen des Knopfes 34 die Kompressionskammer 42 entlüftet wird.

Wie aus Fig. 2 ersichtlich, ist das kegelstumpfförmige vordere Ende 9 des Behälters 8 durch die Verschlußkappe 10 mittels eines Bajonettverschlusses 12 verschließbar, wobei auf dem Behälter 8 vorgesehene Zapfen in das in der Verschlußkappe 10 vorgesehene flache Bajonettgewinde eingreifen. Der Bajonettverschluß 12 ist bis zu einem Druck von mindestens etwa 2 MPa druckdicht. Behälter 8 und Verschlußkappe 10 sind Wegwerfteile und bestehen vorzugsweise aus Polymethylpenten.

Bei der Vorwärtsbewegung des Ausstoßteils 6 unter dem Druck der komprimierten Gase in der Kompressionskammer 42 zu Beginn der Kompressionsphase streicht die eingeschlossene Luft zwischen den Lamellen 26 des Lamellenkörpers 24 und der Innenwand des Behälters 8 vorbei in den rückwärtigen Teil des Behälters 8. Die Lamellen 26 weisen einen Durchmesser von etwa 20 bis 30 mm, vorzugsweise etwa 25 mm, und eine Dicke von etwa 0,5 bis 1,5 mm, vorzugsweise etwa 1 mm auf. Die einzelnen Lamellen 26, 26 sind etwa 1 bis 2 mm voneinander beabstandet. Wenn die vorerste Lamelle 26 bei der Vorkompression mit der Zementsäule in Kontakt steht, ist der Zwischenraum zwischen den Lamellen 26 und der Innenwand des Behälters 8 so klein, daß der Zement nicht zwischen den Lamellen 26 und der Innenwand des Behälters 8 durchgedrückt werden kann und im distalen Teil des Behälters luftfrei komprimiert wird.

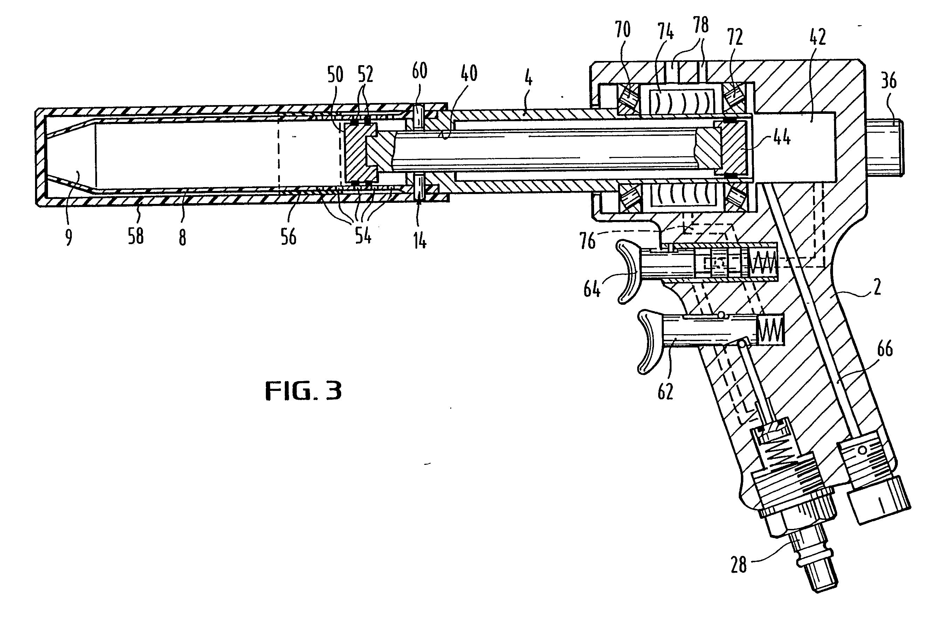

Die Ausführungsform der erfindungsgemäßen Vorrichtung gemäß Fig. 3 unterscheidet sich von der Ausführungsform gemäß Fig. 2 insbesondere durch die Ausbildung der Entlüftungsmittel, der Verschlußkappe und der Drucksteuerung. Ferner ist bei der Ausführungsform gemäß Fig. 3 eine koaxial Rotation des Zements während der Vorkompression möglich.

Gemäß Fig. 3 weist das Ausstoßteil 6 einen vorderen Schieber 50 mit einer Abdichtung, wie Abdichtringen 52 auf, die bündig und gasdicht an den Innenwand des Behälters 8 anliegen. Zumindest der Schieber 50 des Ausstoßteils 6 ist aus einem von Methacrylat nicht angegegriffenen oder oberflächlich angelösten Material, wie Metall, Kunststoff, Keramik oder Teflon® ausgebildet.

Der Behälter 8 weist im hinteren Abschnitt mehrere axial beabstandete Öffnungen 54 auf, über denen eine Manschette 56 verschiebbar ist, die jeweils einen Teil der Öffnungen 54 zumindest zementdicht verschließt.

Die Verschlußkappe 58 ist über nahezu den gesamten Behälter 8 gestülpt und mittels Stiften 60 in den Schnellverschluß 14 eingehakt.

Zur Drucksteuerung sind bei der Ausführungsform gemäß Fig. 3 zwei Ventile 62 und 64 auf eine Entlüftungsleitung 66 vorgesehen, wobei der Aufbau der Drucksteuerung, wie in Fig. 2, lediglich schematisch eingezeichnet ist.

Beim Anmachen und Applizieren des Knochenzementes befindet sich das Ausstoßteil 6 zunächst in der dargestellten hinteren Position. Der Behälter 8 wird mit angerührtem Knochenzement gefüllt und nach distaler Entlüftung die Kappe 58 fest aufgeschraubt. Durch Öffnen des Druckeinlaßventils 62 wird der Kompressionsraum 42 hinter dem Ausstoßteil 6 mit einem Überdruck beaufschlagt. Dadurch wird das als Kolben wirkende Ausstoßteil 6 weiter in den Behälter 8 hineinbewegt. Die über dem eingefüllten Knochenzement überstehende Luft wird durch die Öffnungen 54 seitlich herausgedrückt, wobei die bewegliche Manschette 56 vorher an die Füllhöhe des Zementes angepaßt wurde. Das Ausstoßteil 6 gleitet in der Kolbenführung 40, bis er in schlüssigen Kontakt mit der Oberfläche des Zementes im Behälter 8 kommt.

Nun ist der Zement vollständig und dicht im Behälter 8 eingeschlossen und wird auf einen vorbestimmten Wert komprimiert, der in Abhängigkeit vom zu komprimierenden Zement fest eingestellt wird, vorzugsweise an der (nicht dargestellten) Druckquelle. In diesem Betriebszustand kann durch das zweite Ventil 64 die Kompressionskammer 42 geschlossen werden. Nach Beendigung der Vorkomprimierung, deren Zeitdauer insbesondere von der Viskosität des zu komprimierenden Zementes abhängt, wird die Kompressionskammer 42 durch Betätigung des zweiten Ventiles 64 entlastet, wodurch die Entlüftungsleitung 66 freigegeben wird. Danach wird die Kappe 58 vom Behälter 8 abgenommen, und der Knochenzement kann durch Betätigen des Einlaßventiles 62 und des zweiten Ventiles 64 in dosierter Weise durch das Ausstoßteil 6 ausgeschoben werden. Beim Applizieren des Knochenzementes wird das Ventil 64 nicht fixiert, sondern der Druck wird von Hand auf den gewünschten Wert von z.B. 100 bis 200 kPa eingestellt, der in der Druckmeßvorrichtung 36 als Farbmarkierung ablesbar ist. Nach dem Applizieren wird der geleerte Behälter 8 abgenommen und der Kolben 6 wieder in seine Ausgangsstellung zurückgebracht.

Ferner ist bei der Ausführungsform gemäß Fig. 3 eine Drehvorrichtung zum Rotieren des Zementes vorgesehen. Das Gehäuse 4 ist dabei mittels eines vorderen Schrägrollenlagers 70 und eines hinteren Schrägrollenlagers 72 um seine Längsachse drehbar im Haltegriff 2 befestigt. Auf dem Gehäuse 4 sitzt ein Schaufelrad 74. Zusammen mit einer ventilgesteuerten Treibgaszuführung 76 und Auslaßöffnungen 78 bildet das Schaufelrad 74 eine gasdruckgetriebene Turbine, mit der das Gehäuse 4 einschließlich des Ausstoßteils 6 und dem aufgesetzten Behälter 8 gedreht wird. Zur Steuerung der Drehbewegung kann im Einlaß- oder Auslaßkanal der Turbine ein Ventil vorgesehen sein, das in der Zeichnung zur Vereinfachung weggelassen ist. Die Drehzahl der Turbine beträgt vorzugsweise etwa 1 000 bis 2 000 U/min.

Mit der Vorrichtung gemäß Fig. 3 kann das Vorkomprimieren und Rotieren des Knochenzementes erfindungsgemäß wahlweise nacheinander und/oder gleichzeitig erfolgen. Vorzugsweise wird der Knochenzement zunächst in der vorstehend erläuterten Weise vorkomprimiert. Die Steuerung der Ventile 62 und 64 erfolgt derart, daß die Turbine — während gleichzeitig die Kompressionskammer 42 unter vollem Druck steht — den Behälter 8 in koaxiale Rotation versetzt. Mit der Vorrichtung gemäß Fig. 3 kann der Knochenzement somit gleichzeitig vorkomprimiert und rotiert werden, um eine gezielte radiale Schichtung des Zementes im Behälter 8 zu erreichen. Nach Beendigung der Vorkomprimierung und Rotation wird der Druck abgelassen, und die Applikation erfolgt in der vorstehend erläuterten Weise.

Die Steuerung der Ventile 62 und 64 zum Druckaufbau ist in Fig. 3 lediglich schematisch eingezeichnet. Vorzugsweise weist das Ventil 62 zwei Stellungen auf, wobei in der ersten Stellung die Druckzufuhr in die Kompressionskammer 42 und in der zweiten Stellung die Treibgaszuführung 76 der Turbine freigegeben wird, und wobei in der zweiten Stellung die Zufuhr zur Kompressionskammer 42 gegebenenfalls ebenfalls offen bleiben kann.

Selbstverständlich kann auch bei der Ausführungsform mit Lamellenkörper gemäß den Fig. 1 und 2 die Drucksteuerung, die größere Verschlußkappe und die zusätzliche Drehvorrichtung wie bei der Ausführungsform gemäß Fig. 3 vorgesehen sein.

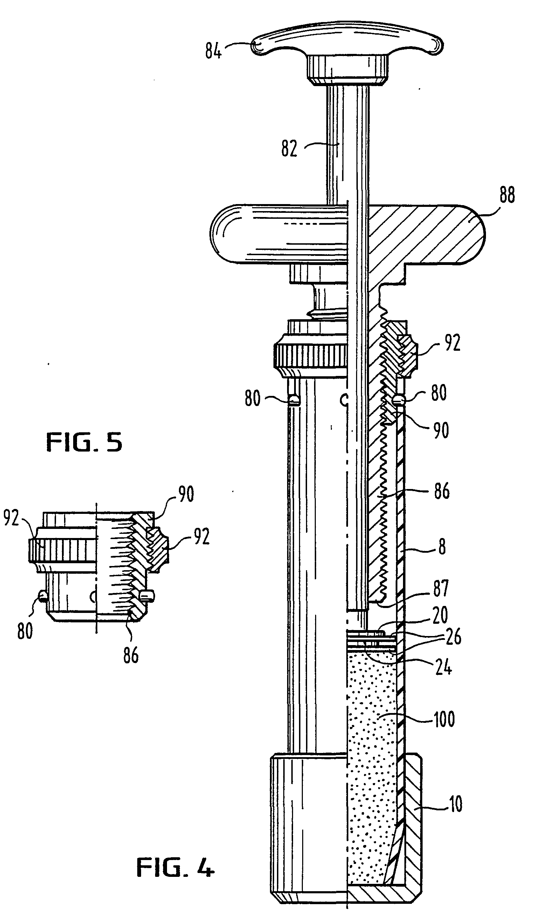

Fig. 4 zeigt im Teilschnitt eine manuelle Ausführungsform der erfindungsgemäßen Vorrichtung zum Applizieren von Knochenzement. Auch bei dieser Ausführungsform sind ein Behälter 8, wie eine Kartusche, zur Aufnahme des Knochenzementes und eine lösbare Verschlußkappe 10 an dessen distalem Ende vorgesehen. Der Behälter 8 ist mit Zylinderstiften 80 am Gehäuse befestigt.

Ferner ist ein Ausstoßteil 82 vorgesehen, dessen vorderes Ende, wie in der Ausführungsform gemäß Fig. 1 und 2, einen Schieber 20 mit darauf aufgesetztem Lamellenkörper 24 mit Lamellen 26 aufweist. Am hinteren Ende des Ausstoßteils 82 ist ein Betätigungsorgan 84 zur manuellen Druckbeaufschlagung vorgesehen. Das Ausstoßteil 82 ist im mittleren Bereich gleitend in einer Gewindestange 84 mit Außengewinde gelagert, an deren hinterem Ende ein Griff 88 angebracht ist. Das Außengewinde der Gewindestange 86 greift in ein Innengewinde eines Führungsteils 90 ein. Über dem Führungsteil 90 ist eine Rändelmutter 92 angeordnet.

Beim Anmachen und Applizieren des Knochenzementes ist die Gewindestange 86 mit Griff 88 zunächst zurückgezogen. Der Behälter 8 wird mit angerührtem Knochenzement gefüllt und an den Zylinderstiften 80 eingehakt. Die Verschlußkappe 10 wird zunächst lose aufgesetzt und erst vollständig festgezogen, wenn das distale Ende des Behälters 8 entlüftet und mit Knochenzement gefüllt ist. Der Knochenzement ist in Fig. 4 mit dem Bezugszeichen 100 gekennzeichnet.

Zunächst wird mit Hilfe des Betätigungsorganes 84 das Ausstoßteil 82 im Behälter 8 nach vorne bewegt, wobei die eingeschlossene Luft an den Lamellen 26 vorbei nach hinten streicht, bis die vorderste Lamelle 26 in schlüssigem Kontakt mit der Zementoberfläche steht (vgl. Fig. 4). Danach wird durch Drehen des Griffs 88 die Gewindestange 86 im Innengewinde des Führungsteils 90 nach vorne bewegt, bis das vordere Ende 87 der Gewindestange 86 in Kontakt mit dem Schieber 20 des Ausstoßteils 82 kommt. Durch Weiterdrehen der Gewindestange 86 wird nun der Knochenzement 100 unter Druck gesetzt und vorkomprimiert.

Auch bei dieser manuellen Druckerzeugung ist der Druck im Knochenzement stufenlos einstellbar. Bei etwa zwei vollen Umdrehungen der Gewindestange 86 im Führungsteil 90, nachdem das vordere Ende 87 mit dem Schieber 20 in Kontakt steht, läßt sich am distalen Ende des Behälters 8 ein Überdruck von etwa 500 bis 600 kPa im Knochenzement erzeugen. Während dieser Phase bleibt der Druck etwa konstant, ohne daß vom Operateur weiter Kraft aufgewandt werden muß.

Nach Beendigung der Vorkompression wird der Knochenzement durch Zurückdrehen der Gewindestange 86 dekomprimiert. Anschließend wird die Verschlußkappe 10 abgeschraubt, und der Knochenzement kann durch Vorschieben des in der Gewindestange 86 gleitenden Ausstoßteils 82 dosiert appliziert werden. Das Vorschieben des Ausstoßteils 82 geschieht ähnlich wie bei einer Spritze, indem das Betätigungsorgan 84 und der Griff 88 umfaßt und gegeneinander gedrückt werden.

Die Länge des Außengewindes der Gewindestange 86 ist derart bemessen, daß auch bei einer Variation der eingefüllten Zementmenge eine vollständige Vorkompression sichergestellt ist.

Sowohl die Vorkompression durch Drehen der Gewindestange 86 im Führungsteil 90 als auch das Applizieren des Zements durch Vorschieben des Ausstoßteils 82 in der Gewindestange 86 können anstelle in manueller Weise auch durch einen Motor, beispielsweise einen Elektromotor durchgeführt werden.

Fig. 5 zeigt im Teilschnitt einen Ausschnitt von Fig. 4 mit der Gewindestange 86 mit Außengewinde, dem Führungsteil 90 mit Innengewinde und der Rändelmutter 92, mit der das Führungsteil 90 gesichert und arretiert werden kann. Ferner sind vier Zylinderstifte 80 vorgesehen, die jeweils um 90° versetzt sind.

Der Außendurchmesser der Gewindestange 86 bzw. der Innendurchmesser des Führungsteils 90 betragen in dieser Ausführungsform vorzugsweise etwa 20 mm.

**Patentansprüche**

1. Vorrichtung zum Mischen und Applizieren von Knochenzement, mit

a) einem zylindrischen Behälter (8) zur Aufnahme des Knochenzementes vor der Applikation,

b) einem im Behälter (8) beweglichen, zementdicht gegen den Behälter (8) abschließbaren Ausstoßteil (6),

c) im Behälter (8) oder am Ausstoßteil (6) vorgesehenen Entlüftungsmitteln (24, 54) zum Entweichen der den Knochenzement überstehenden Luft bei einer Vorwärtsbewegung des Ausstoßteils (6),

d) einer lösbaren Verschlußkappe (10; 58), durch die der Behälter (8) an seinem distalen Ende dicht abschließbar ist, und

e) einer pneumatischen Druckerzeugungsvorrichtung (42) zum Vorkomprimieren des Knochenzements im Behälter (8) und zu dessen Applikation, durch welche der beim Vorkomprimieren und Applizieren des Knochenzements ausgeübte Druck exakt stufenlos steuerbar und/oder konstant einstellbar ist.

2. Vorrichtung zum Mischen und Applizieren von Knochenzement mit

a) einem zylindrischen Behälter (8) zur Aufnahme des Knochenzementes vor der Applikation,

b) einem im Behälter (8) beweglichen, zementdicht gegen den Behälter (8) abschließbaren Ausstoßteil (82),

c) im Behälter (8) oder am Ausstoßteil (82) vorgesehenen Entlüftungsmitteln (24, 54) zum Entweichen der den Knochenzement überstehenden Luft bei einer Vorwärtsbewegung des Ausstoßteils (82),

d) einer lösbaren Verschlußkappe (10; 58), durch die der Behälter (8) an seinem distalen Ende dicht abschließbar ist, und

e) einer manuell oder durch einen Motor betätigbaren Druckerzeugungsvorrichtung (86) zum Vorkomprimieren des Knochenzements im Behälter (8) und zu dessen Applikation, wobei die Vorkompression mit stufenlos oder konstant einstellbarem Druck und die Applikation mit exakt stufenlos steuerbarem Druck durchgeführt wird.

3. Vorrichtung nach Anspruch 1 oder 2, dadurch gekennzeichnet, daß der Knochenzement im Behälter (8) mit Drücken von etwa 200 kPa bis zu etwa 2 MPa beaufschlagt wird.

4. Vorrichtung nach einem der Ansprüche 1 bis 3, dadurch gekennzeichnet, daß die Entlüftungsmittel durch Längsschlitze und/oder durch mehrere, in Axialrichtung beabstandete Ventile oder Öffnungen (54) im Behälter (8) ausgebildet werden.

5. Vorrichtung nach Anspruch 4, gekennzeichnet durch eine in Axialrichtung auf dem Behälter (8) verschiebbare Manschette (56).

6. Vorrichtung nach einem der Ansprüche 1 bis 3, gekennzeichnet durch einen auf das Ausstoßteil (6) aufgesetzten Lamellenkörper (24) mit mindestens einer flexiblen, kreisförmigen Lamelle (26), vorzugsweise aus Teflon, als Entlüftungsmittel.

7. Vorrichtung nach einem der Ansprüche 1 bis 6, dadurch gekennzeichnet, daß der Behälter (8) als Einwegbehälter ausgebildet ist, der mit einem Schnellverschluß (14) an einem Gehäuse (4) befestigbar ist.

8. Vorrichtung nach einem der Ansprüche 1 bis 7, dadurch gekennzeichnet, daß die Verschlußkappe (10; 58) mittels eines steigenden Bajonettverschlusses (12) entlüftbar und druckdicht auf den Behälter (8) aufsetzbar ist.

9. Vorrichtung nach einem der Ansprüche 1 oder 3 bis 8, gekennzeichnet durch eine Kompressionskammer (42) mit einer Zuführung für komprimierte Gase, wie Druckluft zum Beaufschlagen des Ausstoßteils (6), wobei der auf das Ausstoßteil (6) wirkende Druck vorzugsweise mittels mindestens eines Druckeinlaßventils und/oder Druckablaßventils (30, 32, 34; 62, 64) steuerbar ist.

10. Vorrichtung nach einem der Ansprüche 2 bis 8, gekennzeichnet durch eine drehbare Gewindestange (86) zur Druckerzeugung.

11. Vorrichtung nach Anspruch 10, dadurch gekennzeichnet, daß die Gewindestange manuell oder durch einen Motor betätigt wird.

12. Vorrichtung nach einem der Ansprüche 1 bis 11, dadurch gekennzeichnet, daß das distale Ende (9) des zylindrischen Behälters (8) als offener Kegelstumpf oder als Verjüngung mit ovaler Öffnung ausgebildet ist.

13. Vorrichtung nach einem der Ansprüche 1 bis 12, dadurch gekennzeichnet, daß die Öffnung des distalen Endes (9) des Behälters (8) während der Applikation des Knochenzements mittels einer Blende oder eines variablen Aufsatzes veränderlich ist.

14. Vorrichtung nach einem der Ansprüche 1 bis 13, dadurch gekennzeichnet, daß der Behälter (8) aus mindestens zwei in Axialrichtung hintereinander angeordneten Zylindern besteht, die mittels eines Adapters koppelbar sind.

15. Vorrichtung nach einem der Ansprüche 1 bis 14, gekennzeichnet durch eine Drehvorrichtung (70, 72) zum koaxialen Drehen des mit Knochenzement gefüllten und unter Druck stehenden Behälters (8).

16. Vorrichtung nach Anspruch 15, gekennzeichnet durch eine gasdruckgetriebene Turbine (74, 76, 78) zum Antrieb der Drehvorrichtung (70, 72) und dadurch, daß mindestens eines der Ventile (62, 64) als Umwegventil ausgebildet ist, durch dessen Betätigung die Turbine (74, 76, 78) mit der Gaszuführung verbindbar ist.

17. Vorrichtung nach Anspruch 15, gekennzeichnet durch einen Motor zum Abtrieb der Drehvorrichtung (70, 72).

18. Verfahren zum Mischen und Applizieren von Knochenzement mit einer Vorrichtung nach einem der Ansprüche 1, 3 bis 9 und 12 bis 17, bei dem der Knochenzement zuerst mit exakt stufenlos steuerbarem oder mit konstant einstellbarem Druck ohne Lufteinschluß im Knochenzement vorkomprimiert wird, wobei die den Knochenzement überstehende Luft bei einer Vorwärtsbewegung des Ausstoßteils (6) durch im Behälter (8) zur Aufnahme des Knochenzements oder durch am Ausstoßteil (6) vorgesehene Entlüftungsmittel (24, 54) entweicht, danach der Knochenzement dekomprimiert und anschließend mit exakt stufenlos steuerbarem, niedrigerem Druck appliziert wird, wobei die Vorkompression und die Applikation mit pneumatischer Druckbeaufschlagung durchgeführt werden.

19. Verfahren zum Mischen und Applizieren von Knochenzement mit einer Vorrichtung nach einem der Ansprüche 2 bis 8 und 10 bis 17, bei dem der Knochenzement zuerst mit stufenlos oder konstant einstellbarem Druck ohne Lufteinschluß im Knochenzement vorkomprimiert wird, wobei die den Knochenzement überstehende Luft bei einer Vorwärtsbewegung das Ausstoßteils (82) durch im Behälter (8) zur Aufnahme des Knochenzements oder durch am Ausstoßteil (82) vorgesehene Entlüftungsmittel (24; 54) entweicht, danach der Knochenzement dekomprimiert und anschließend mit exakt stufenlos steuerbarem, niedrigerem Druck appliziert wird, wobei die Vorkompression und die Applikation mit manueller oder motorbetätigter Druckbeaufschlagung durchgeführt werden.

20. Verfahren nach Anspruch 18 oder 19, dadurch gekennzeichnet, daß eine poröse Fraktion und eine dichtere Fraktion des Knochenzements vor der Kompression in einem Behälter übereinander geschichtet werden oder daß der Knochenzement vor seiner Applikation rotiert wird, um eine radiale Schichtung seiner Fraktionen im Behälter auszubilden.

## Revendications

1. Dispositif pour le mélange et l'application de ciment osseux, comprenant

a) un réservoir cylindrique (8) pour recevoir le ciment osseux avant l'application,

b) une pièce d'expulsion (6) qui est déplaçable dans le réservoir (8) et peut obturer le réservoir (8) de manière étanche au ciment,

c) des moyens de désaération (24, 54) prévus dans le réservoir (8) ou sur la pièce d'expulsion (6) pour dégager l'air situé au-dessus du ciment osseux, au cours d'un déplacement vers l'avant de la pièce d'expulsion (6),

d) un capuchon de fermeture détachable (10, 58), par lequel le réservoir (8) peut être fermé de manière étanche à son extrémité distale, et

e) un dispositif de production de pression pneumatique (42) qui est prévu pour la précompression du ciment osseux dans le réservoir (8) et pour son application et par lequel la pression exercée au cours de la précompression et de l'application du ciment osseux peut être réglée de manière exacte et progressive et/ou être ajustée à une valeur constante.

2. Dispositif pour le mélange et l'application de ciment osseux comprenant

a) un réservoir cylindrique (8) pour recevoir le ciment osseux avant l'application,

b) une pièce d'expulsion (82) qui est déplaçable dans le réservoir (8) et peut obturer le réservoir (8) d'une manière étanche au ciment,

c) des moyens de désaération (24, 54) prévus dans le réservoir (8) ou sur la pièce d'expulsion (82) pour dégager l'air situé au-dessus du ciment ossseux, au cours d'un mouvement vers l'avant de la pièce d'expulsion (82),

d) un capuchon de fermeture détachable (10, 58), par lequel le réservoir (8) peut être fermé de manière étanche à son extrémité distale, et

e) un dispositif de production de pression (86) qui peut être actionné manuellement ou par un moteur en vue de la précompression cu ciment osseux dans le réservoir (8) et de son application, la précompression étant effectuée à une pression adjustable de manière progressive ou à une valeur constante et l'application étant effectuée avec une pression réglable de manière exacte et progressive.

3. Dispositif suivant l'une des revendications 1 et 2, caractérisé en ce que le ciment osseux dans le réservoir (8) est sollicité par des pressions d'environ 200 kPa jusqu'à environ 2 MPa.

4. Dispositif suivant l'une des revendications 1 à 3, caractérisé en ce que les moyens de désaération sont réalisés dans le réservoir (8) par des fentes longitudinales et/ou par plusieurs soupapes ou orifices (54) disposés à distance suivant la direction axiale.

5. Dispositif suivant la revendication 4, caractérisé par un manchon (56) qui peut être déplacé en direction axiale sur le réservoir (8).

6. Dispositif suivant l'une des revendications 1 à 3, caractérisé par un corps à lamelles (24) qui est monté sur la pièce d'expulsion (6) et qui est pourvu d'au moins une lamelle circulaire (26) flexible, de préférence en Teflon, comme moyen de désaération.

7. Dispositif suivant l'une des revendications 1 à 6, caractérisé en ce que le réservoir (8) est réalisé sous la forme d'un réservoir à usage unique qui peut être fixé sur un boîtier (4) à l'aide d'une fermeture rapide (14).

8. Dispositif suivant l'une des revendications 1 à 7, caractérisé en ce que le capuchon de fermeture (10, 58) peut être désaéré au moyen d'une fermeture à baïonnette (12) croissante et en ce qu'elle peut être montée sur le réservoir (8) d'une manière étanche à la pression.

9. Dispositif suivant l'une des revendications 1 ou 3 à 8, caractérisé par une chambre de compression (42) avec une amenée pour des gaz comprimés, comme de l'air sous pression, pour solliciter la pièce d'expulsion (6), la pression agissant sur la pièce d'expulsion (6) pouvant être réglée de préférence au moyen d'au moins une soupape d'admission de pression et/ou d'une soupape de sortie de pression (30, 32, 34; 62, 64).

10. Dispositif suivant l'une des revendications 2 à 8, caractérisé par une tige filetée (86) capable de tourner, pour la production de la pression.

11. Dispositif suivant la revendication 10, caractérisé en ce que la tige filetée est actionnée manuellement ou par un moteur.

12. Dispositif suivant l'une des revendications 1 à 11, caractérisé en ce que l'extrémité distale (9) du réservoir cylindrique (8) est réalisée sous la forme d'un tronc de cône ouvert ou sous la forme d'un amincissement à ouverture ovale.

13. Dispositif suivant l'une des revendications 1 à 12, caractérisé en ce que l'ouverture de l'extrémité distale (9) du réservoir (8) peut être modifiée pendant l'application du ciment osseux au moyen d'un écran ou d'un chapeau variable.

14. Dispositif suivant l'une des revendications 1 à 13, caractérisé en ce que le réservoir (8) est constitué d'au moins deux cylindres qui sont disposés l'un derrière l'autre en direction axiale et qui peuvent être accouplés au moyen d'un adaptateur.

15. Dispositiff suivant l'une des revendications 1 à 14, caractérisé par un dispositif de rotation (70, 72) destiné à la rotation coaxiale du réservoir (8) rempli de ciment osseux et se trouvant sous pression.

16. Dispositif suivant la revendication 15, caractérisé par une turbine propulsée par du gaz sous pression (74, 76, 78) pour l'entraînement du dispositif de rotation (70, 72) et en ce qu'au moins une des soupapes (62, 64) est réalisée sous la forme d'une soupape de détournement par l'actionnement de laquelle la turbine (74, 76, 78) peut être mise en communication avec l'amenée de gaz.

17. Dispositif suivant la revendication 15, caractérisé par un moteur pour l'entraînement du dispositif de rotation (70, 72).

18. Procédé pour le mélange et l'application de ciment osseux à l'aide d'un dispositif suivant l'une des revendications 1, 3 à 9 et 12 à 17, dans lequel le ciment osseux est tout d'abord précomprimé à une pression réglable de manière exacte et progressive ou adjustable à une valeur constante, sans inclusion d'air dans le ciment osseux, l'air situé au-dessus du ciment osseux étant, au cours d'un mouvement vers l'avant de la pièce d'expulsion (6), dégagé par des moyens de désaération (24, 54) prévus dans le réservoir (8) destiné à recevoir le ciment osseux ou sur la pièce d'expulsion (6), le ciment osseux étant après cela décomprimé et appliqué ensuite avec une pression plus faible qui peut être réglée de manière exacte, progressivement, la précompression et l'application étant effectuées à l'aide d'une alimentation en pression pneumatique.

19. Procédé pour le mélange et l'application de ciment osseux à l'aide d'un dispositif suivant l'une des revendications 2 à 8 et 10 à 17, dans lequel le ciment osseux est tout d'abord précomprimé à une pression adjustable progressivement ou à valeur constante, sans inclusion d'air dans le ciment osseux, l'air situé au-dessus du ciment osseux étant, au cours d'un déplacement vers l'avant de la pièce d'expulsion (82), dégagé par des moyens de désaération (24, 54) prévus dans le réservoir (8) destiné à recevoir le ciment osseux ou sur la pièce d'expulsion (82) le ciment osseux étant après cela décomprimé et ensuite appliqué avec une pression plus basse qui peut être réglée progressivement de manière exacte, la précompression et l'application étant effectuées à l'aide d'une alimentation en pression manuelle ou actionnée par moteur.

20. Procédé suivant l'une des revendications 18 et 19, caractérisé en ce qu'une fraction poreuse et une fraction plus dense du ciment osseux sont disposées en couches l'une au-dessus de l'autre dans un réservoir, avant la compression, ou en ce que le ciment osseux est entraîné en rotation avant son application, pour réaliser une disposition en couches radiale de ses fractions dans le réservoir.

**Claims**

1. A device for mixing and applying bone cement, comprising
   a) a cylindrical container (8) for receiving the bone cement prior to its application,
   b) an ejector member (6) that is movable in the container (8) and seals against the said container (8) in a cement-tight manner,
   c) vent means (24, 54) provided in the container (8) or at the ejector member (6) to let the air above the bone cement escape as the ejector member (6) moves forward,
   d) a removable closure cap (10; 58), by which the container (8) can be tightly closed at its distal end, and
   e) a pneumatic pressure generating means (42) for prepressurising the bone cement in the container (8) and for applying said bone cement, by which means the pressure exerted during the prepressurising and applying of the one cement is precisely and infinitely controllable and/or adjustable to a constant value.

2. A device for mixing and applying bone cement comprising
   a) a cylindrical container (8) for receiving the bone cement prior to its application,
   b) an ejector member (82) that is movable in the container (8) and seals against the said container (8) in a cement-tight manner,
   c) vent means (24, 54) provided in the container (8) or at the ejector member (82) to let the air above the bone cement escape as the ejector member (82) moves forward,
   d) a removable closure cap (10; 58), by which the container (8) can be tightly closed at its distal end, and
   e) a pressure generating means (86), which is operable manually or via a motor, for prepressurising the bone cement in the container (8) and for applying said bone cement, wherein precompression is carried out under infinitely adjustable pressure or pressure adjustable to a constant value and application is carried out under precisely and infinitely controllable pressure.

3. The device according to claim 1 or 2, characterised in that the bone cement is placed under pressures of about 200 kPa to about 2 MPa in the container (8).

4. The device according to any of claims 1 to 3, characterised in that the vent means are formed by longitudinal slots and/or a plurality of axially spaced valves or openings (54) in the container (8).

5. The device according to claim 4, characterised by a sleeve member (56) that is axially slidable on the container (8).

6. The device according to any of claims 1 to 3, characterised by a lamellar member (24) mounted on the ejector member (6) and having at least one flexible, circular lamella (26), preferably made of teflon, as the vent means.

7. The device according to any of claims 1 to 6, characterised in that the container (8) is disposable and can be secured to a casing (4) by means of a fast closure (14).

8. The device according to any of claims 1 to 7, characterised in that the closure cap (10; 58) can be vented and mounted in a pressure-tight manner onto the container (8) by means of a slanted bayonet closure (12).

9. The device according to any of claims 1 or 3 to 8, characterised by a compression chamber (42) having a supply for compressed gases, such as pressurised air, for exerting pressure on the ejector member (6) the pressure exerted on the ejector member (6) preferably being controlled by means of at least one pressure inlet valve and/or pressure outlet valve (30, 32, 34; 62, 64).

10. The device according to any of claims 2 to 8, characterised by a rotatable threaded rod (86) for generating pressure.

11. The device according to claim 10, characterised in that the threaded rod is operated either manually or via a motor.

12. The device according to any of claims 1 to 11, characterised in that the distal end (9) of the cylindrical container (8) is an open truncated cone or a taper with an oval opening.

13. The device according to any of claims 1 to 12, characterised in that the opening of the distal end (9) of the container (8) can be varied by means of a screening member or variable mounting member during the application of the bone cement.

14. The device according to any of claims 1 to 13, characterised in that the container (8) is comprised of at least two cylinders that are arranged in axial succession and can be connected by means of an adapter.

15. The device according to any of claims 1 to 14, characterised by a rotating apparatus (70, 72) for coaxially rotating the pressurised container (8) filled with bone cement.

16. The device according to claim 15, characterised by a gas pressure driven turbine (74, 76, 78) for driving the rotating apparatus (70, 72) and in that at least one of the valves (62, 64) is a bypass valve whose operation permits the connection of the turbine (74, 76, 78) to the gas supply.

17. The device according to claim 15, characterised by a motor for driving the rotating apparatus (70, 72).

18. A process for mixing and applying bone cement using a device according to any of claims 1, 3 to 9 and 12 to 17, wherein the bone cement is first precompressed under precisely and infinitely controllable pressure or pressure adjustable to a contant value without air inclusion in said bone cement, the air above the bone cement escaping through vent means (24, 54) provided in the container (8) for receiving the bone cement or at the ejector member (6) when said ejector member (6) moves forward, the bone cement is then decompressed and subsequently applied under precisely and infinitely controllable lower pressure, precompression and application being carried out under pneumatic pressure.

19. A process for mixing and applying bone cement using a device according to any of claims 2 to 8 and 10 to 17, wherein the bone cement is first precompressed under inifinitely adjustable pressure or pressure adjustable to a constant value without air inclusion in said bone cement, the air above the bone cement escaping through vent means (24; 54) provided in the container (8) for receiving the bone cement or at the ejector member (82) when said ejector member (82) moves forward, the bone cement is then decompressed and subsequently applied under precisely and infinitely controllable lower pressure, precompression and application being carried out under manual or engine-driven pressure.

20. The process according to claim 18 or 19, characterised in that prior to compression a porous fraction and a more dense fraction of the bone cement are placed on top of one another in a container or in that the bone cement is rotated prior to its application in order to form a radial layer arrangement of its fractions in the container.

# FIG. 1

FIG. 2

EP 0 170 120 B1

FIG. 3

FIG. 5

FIG. 4